# EUROPEAN PATENT SPECIFICATION

(11) **EP 1 142 534 B1**
(45) Date of publication and mention of the grant of the patent: **14.07.2004**
(21) Application number: 01303242.0
(22) Date of filing: 05.04.2001
(51) Int. Cl.: A61B 5/15

(54) **Blood sampling device with adjustable puncture depth**
Blutentnahmevorrichtung mit verstellbarer Punktionstiefe
Dispositif de prélévement sanguin avec une profondeur de ponction reglable

(30) Priority: 06.04.2000 PL 33953000
(43) Date of publication of application: 10.10.2001
(73) Proprietor: PZ HTL S.A., 03-230 Warszawa (PL)
(72) Inventor: Wyszogrodzki, Wojciech, Warszawa (PL); Rutynowski, Wlodzimierz, 00-770 Warszawa (PL)
(74) Representative: Silverman, Warren

(56) References cited:
- EP-A- 0 885 590
- EP-A- 0 904 790
- US-A- 5 356 420
- US-A- 5 613 978

## Description

The present invention relates to a sampling device suitable, in particular, for puncturing skin of a patient in order to take a blood sample for diagnostic purposes.

US patent 5,356,420 discloses a puncturing device comprising a sleeve and a push element positioned at one end of the sleeve. The other end of the sleeve is open. Within the sleeve there is a slidably mounted piston, which at the end closer to the push element terminates in a pusher, and which at the end closer to the open end of the sleeve terminates in a puncturing tip. Inside the sleeve, between the end face of the push element and the piston, a driving spring is located. A return spring is located between the piston and the open end of the sleeve. Wings are located on the outer circumference of the piston which rest on an internal projection of the sleeve.

A similar device is disclosed in US 5,613,978. Here, a device for achieving different depths of skin puncture which comprises an adjustable outer cover for varying depths of puncture is described.

According to the present invention, there is provided a sampling device of a type which comprises a sleeve, a push element mounted at one end of the sleeve, a piston having a fin and a puncturing tip extending from the fin, the piston being slidably mounted in the sleeve, a drive spring positioned between the end face of the push element and the piston, wherein, at the other end of the sleeve, an adjusting ring is mounted having an opening for receiving the puncturing tip. The adjusting ring has two inner semi-annular limiting members each having a substantially oblique abutment surface against which the fin of the piston abuts during use.

The sampling device of the invention is a puncturing device, primarily, but not only, intended for puncturing the skin of a patient and is generally referred to hereinafter as a puncturing device.

Preferably the outer surface of the adjusting ring is provided with a marker, and on the outer surface of the sleeve is provided with a scale for indicating the depth of the puncture.

For a better understanding of the invention, and to show how the same may be carried into effect, reference will now be made, by way of example, to the accompanying drawings, in which:-
Fig. 1 shows a longitudinal section through a puncturing device with the apparatus for regulating the depth of the puncture before use in accordance with the first aspect of the present invention;
Fig. 2 shows a perspective view of an apparatus for regulating puncture depth in accordance with the second aspect of the present invention;
Fig. 3 shows a side view of the puncturing device of Fig 1 with a scale for indicating the depth of the puncture in use;
Fig. 4 shows a longitudinal section through the puncturing device with the apparatus for regulating depth of the puncture of Fig. 1, after use.

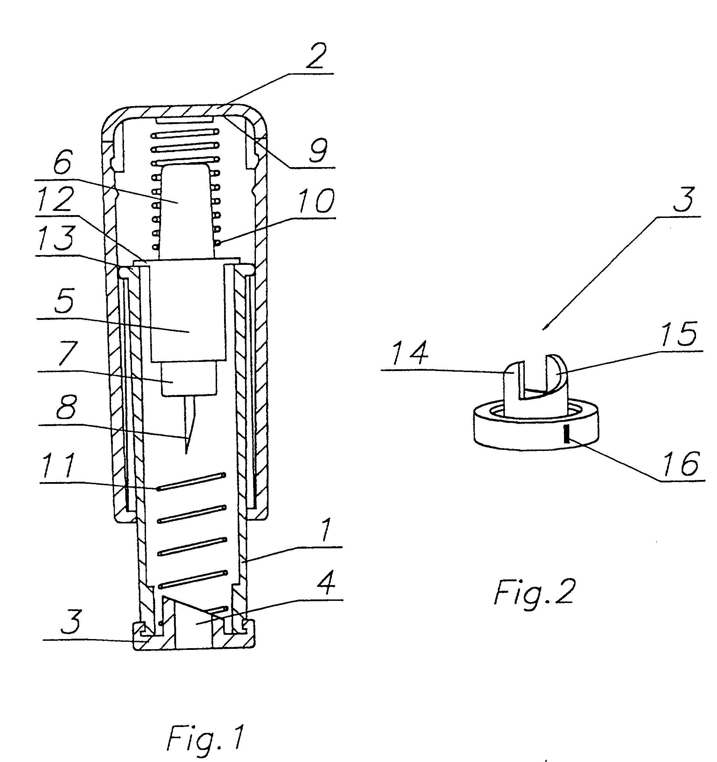

The puncturing device shown in Fig. 1 comprises a sleeve 1 and a push element 2 mounted at one end of the sleeve 1 and surrounding substantially the entire length of the sleeve 1. At the other end of the sleeve 1 is an adjustment ring 3 having an opening 4.

A slidably mounted piston 5 is located within the sleeve. At the end closer to the push element 2, the piston has a pusher 6, and at the end closer to the adjustment ring 3, the piston has a fin 7 with a puncturing tip 8. A drive spring 10 is positioned inside the device, between the end face 9 of the push element 2 and the piston 5. A return spring 11 is positioned inside the sleeve 1, between the piston 5 and the adjusting ring 3. In this particular embodiment the piston 5 has outwardly directed wings 12 at the upper section, which rest on the upper edge 13 of the sleeve 1.

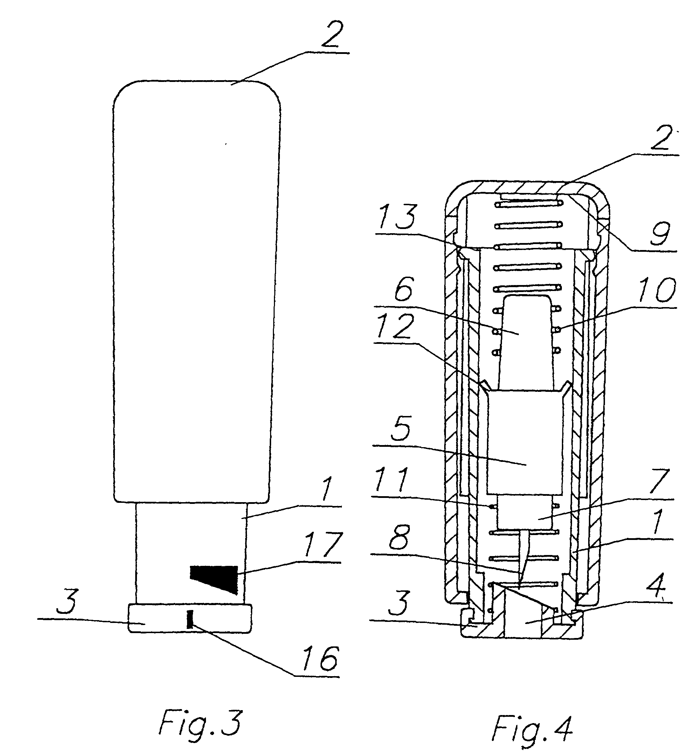

A perspective view of the adjusting ring 3 is shown in Fig. 2. The adjusting ring 3 has an opening 4 for receiving the puncturing tip 8. It also has, directed inwardly into the sleeve 1, two half-ringed, oblique limiting members 14, 15, which allow smooth adjustment of the depth to which the puncturing tip 8 penetrates the skin of a patient. Alternatively, the limiting members may be arranged as steps, instead of as smooth oblique limiting members 14, 15, for regulation of the depth of penetration in a stepwise manner. As shown in Fig. 3, the outer surface of the adjusting ring 3 is provided with a marker 16, and the outer surface of the sleeve 1 is provided with a scale 17 placed in front of marker 16, to indicate the depth of the puncturing tip 8, in use.

The device according to the present invention operates in the following manner. The position of the elements of the device before use is shown in fig. 1. As a result of the pressure applied by the drive spring 10, the wings 12 of the piston 5 rest on the upper edge 13 of the sleeve 1. In this way, the piston 5 with puncturing tip 8 is held in a first stable position. Pressing the push element 2 causes compression of the drive spring 10 to the point that the end face 9 of the push element 2 rests on pusher 6 of the piston 5.

Applying further pressure to the push element 2 causes the wings 12 of the piston 5 to break off, and the drive spring 10 driving the piston 5 causes the fin 7 of piston 5 to hit the members 14, 15 thus limiting the extension of the projecting tip through the opening 4 of the adjusting ring 3, and thus limiting the depth of the puncture of the patient's skin. Subsequently, the return spring 11 withdraws the piston 5 with the puncturing tip 8, which then assumes a second stable position inside the sleeve 1.

The depth of the puncture of the patient's skin is adjusted by turning the adjusting ring 3 about the axis of the device, preferably with use of a ratchet mechanism. In this way the position of the half-ringed, oblique limiting members 14, 15 may be altered in relation to fin 7 of the piston 5 and thus, after the fin 7 of the piston 5 hits the limiting members, the depth to which the puncturing tip 8 penetrates the patient's body is also altered.

The position of the elements of the device after use is shown in Fig. 4. The broken off wings 12 of the piston 5 are shown. Reuse of the device once the wings 12 have been broken off is not possible.

Although the apparatus for a sampling device, for regulating the depth of puncture of a sampling device, has been described with reference to a sampling device where the piston has breakable wings, it will be appreciated that such an apparatus can be used with devices without such wings. Alternatively, the sleeve, rather than the piston, may have breakable wings on which an external projection of the piston rests prior to use. Or, the piston may have a detachable flange on its outer surface that rests on the first end of the sleeve. Such alternative arrangements of breakable wings are described in co-pending European Patent application filed on 4^{th} April 2001, published as EP-A- 1 247 489.

## Claims

1. A sampling device comprising a sleeve (1), a push element (2) mounted at a first end of the sleeve (1), a piston (5) having a fin (7) and a puncturing tip (8) extending from the fin (7), the piston (5) being slidably mounted in the sleeve (1), a drive spring (10) positioned between the end face (9) of the push element (2) and the piston (5), wherein at the other end of the sleeve (1) an adjusting ring (3) is mounted having an opening (4) for receiving the puncturing tip (8), **characterised in that** the adjusting ring has two inner semi-annular limiting members (14, 15) each having a substantially oblique abutment surface against which the fin (7) of the piston (5) abuts during use.

2. A sampling device as claimed in claim 1, wherein the abutment surface of each limiting member (14, 15) is a substantially smooth curve.

3. A sampling device as claimed in claim 1, wherein the abutment surface of each limiting member (14, 15) comprises a series of steps.

4. A sampling device according to my preceding claim wherein the side surface of the adjusting ring (3) is provided with a marker (16) and the outer surface of the sleeve (1) is provided with a scale (17), for indicating the depth of the puncture of the sampling device, in use.

5. A sampling device according to any preceding claim wherein the sleeve (1) has breakable wings directed inwardly and the piston (5) has an external projection which rests on the wings.

6. A sampling device according to any of claims 1 to 4 wherein the piston (5) has breakable wings (12) or a detachable flange which rests on the upper edge (13) of sleeve (1).

7. A sampling device according to any of claims 1 to 4 wherein the sleeve (1) comprises a washer having breakable wings and an outer ring, the washer being positioned at the first end of the sleeve (1) and the piston (5) has an external projection which rests-on the wings.

## Patentansprüche

1. Blutentnahmevorrichtung, aufweisend eine Hülse (1), ein Schubelement (2), das an einem ersten Ende der Hülse (1) angebracht ist, einen Kolben (5) mit einem Leitabschnitt (7) und einer Einstechspitze (8), die sich von dem Leitabschnitt (7) aus erstreckt, wobei der Kolben (5) verschiebbar in der Hülse (1) angebracht ist, wobei eine Antriebsfeder (10) zwischen der Endfläche (9) des Schubelements (2) und dem Kolben (5) positioniert ist, wobei am anderen Ende der Hülse (1) ein Einstellring (3) angebracht ist, welcher eine Öffnung (4) aufweist, um die Einstechspitze (8) aufzunehmen, **dadurch gekennzeichnet, dass** der Einstellring zwei innere halbringförmige Begrenzungselemente (14, 15) aufweist, von denen jedes eine im Wesentlichen schräge Anlageoberfläche aufweist, gegen die das Leitelement (7) des Kolbens (5) während der Verwendung anliegt.

2. Blutentnahmevorrichtung nach Anspruch 1, wobei die Anlageoberfläche jedes Begrenzungselements (14, 15) eine im Wesentlichen glatte Kurve ist.

3. Blutentnahmevorrichtung nach Anspruch 1, wobei die Anlageoberfläche jedes Begrenzungselements (14, 15) eine Folge von Stufen aufweist.

4. Blutentnahmevorrichtung nach einem der vorstehenden Ansprüche, wobei die Seitenoberfläche des Einstellrings (3) mit einer Markierung (16) versehen ist, und die äußere Oberfläche der Hülse (1) mit einer Skala (17), um die Tiefe des Einstichs der Blutentnahmevorrichtung während der Verwendung anzuzeigen.

5. Blutentnahmevorrichtung nach einem der vorstehenden Ansprüche, wobei die Hülse (1) zerbrechbare Flügel aufweist, die nach innen gerichtet sind, und der Kolben (5) einen äußeren Vorsprung aufweist, der auf den Flügeln aufliegt.

6. Blutentnahmevorrichtung nach einem der Ansprüche 1 bis 4, wobei der Kolben (5) zerbrechbare Flügel (12) oder einen entfernbaren Flansch aufweist, der auf der oberen Kante (13) der Hülse (1) aufliegt.

7. Blutentnahmevorrichtung nach einem der Ansprüche 1 bis 4, wobei die Hülse (1) eine Beilagscheibe mit abbrechbaren Flügeln und einen äußeren Ring aufweist, wobei die Beilagscheibe an dem ersten Ende der Hülse (1) positioniert ist und der Kolben (5) einen externen Vorsprung aufweist, der auf den Flügeln aufliegt.

## Revendications

1. Dispositif de prélèvement comportant un manchon (1), un élément de poussée (2) monté au niveau d'une première extrémité du manchon (1), un piston (5) ayant un stabilisateur (7) et une pointe de perforation (8) s'étendant à partir du stabilisateur (7), le piston (5) étant monté de manière coulissante dans le manchon (1), un ressort d'entraînement (10) étant positionné entre la face d'extrémité (9) de l'élément de poussée (2) et le piston (5), où un anneau d'ajustement (3) étant monté au niveau de l'autre extrémité du manchon (1), ayant une ouverture (4) pour recevoir la pointe de perforation (8),
**caractérise en ce que** l'anneau d'ajustement a deux éléments de limitation semi-annulaires intérieurs, (14,15), ayant chacun une surface de butée sensiblement oblique contre laquelle le stabilisateur (7) du piston (5) vient en butée pendant utilisation.

2. Dispositif de prélèvement selon la revendication 1, dans lequel la surface de butée de chaque élément de limitation (14,15) est une courbe sensiblement lisse.

3. Dispositif de prélèvement selon la revendication 1, dans lequel la surface de butée de chaque élément de limitation (14, 15) comporte une série de gradins.

4. Dispositif de prélèvement selon l'une quelconque des revendications précédentes, dans lequel la surface latérale de l'anneau d'ajustement (3) est munie d'un indicateur (16), et la surface extérieure du manchon (1) est munie d'une échelle (17), pour indiquer la profondeur de la perforation du dispositif de prélèvement, en utilisation.

5. Dispositif de prélèvement selon l'une quelconque des revendications précédentes, dans lequel le manchon (1) a des ailes pouvant être brisées dirigées vers l'intérieur, et le piston (5) a une saillie externe qui s'appuie sur les ailes.

6. Dispositif de prélèvement selon l'une quelconque des revendications 1 à 4, dans lequel le piston (5) a des ailes pouvant être brisées (12) ou un rebord détachable qui s'appuie sur le bord supérieur (13) du manchon (1).

7. Dispositif de prélèvement selon l'une quelconque des revendications 1 à 4, dans lequel le manchon (1) comporte une rondelle ayant des ailes pouvant être brisées et un anneau extérieur, la rondelle étant positionnée au niveau de la première extrémité du manchon (1), et le piston (5) comporte une saillie externe qui s'appuie sur les ailes.
